(19) Europäisches Patentamt
European Patent Office
Office européen des brevets

(11) **EP 0 668 263 B1**

(12) **EUROPÄISCHE PATENTSCHRIFT**

(45) Veröffentlichungstag und Bekanntmachung des Hinweises auf die Patenterteilung:
**27.08.1997 Patentblatt 1997/35**

(51) Int Cl.$^6$: **C07C 205/06**, C07C 201/08

(21) Anmeldenummer: **95101348.1**

(22) Anmeldetag: **01.02.1995**

(54) **Verfahren zur adiabatischen Herstellung von Mononitrotoluolen**

Process for the adiabatic preparation of mononitrotoluenes

Procédé pour la préparation adiabatique de mononitrotoluènes

(84) Benannte Vertragsstaaten:
**BE DE ES FR GB IT NL PT SE**

(30) Priorität: **14.02.1994 DE 4404614**
**25.03.1994 DE 4410417**

(43) Veröffentlichungstag der Anmeldung:
**23.08.1995 Patentblatt 1995/34**

(73) Patentinhaber: **BAYER AG**
**51368 Leverkusen (DE)**

(72) Erfinder:
• **König, Bernd-Michael Dr.**
**D-40227 Düsseldorf (DE)**
• **Judat, Helmut Dr.**
**D-40764 Langenfeld (DE)**
• **Blank, Heinz Ulrich Dr.**
**D-51519 Odenthal-Glöbusch (DE)**

(56) Entgegenhaltungen:
CA-A- 1 297 132      US-A- 2 256 999
US-A- 4 021 498      US-A- 4 091 042

## Beschreibung

Die vorliegende Erfindung betrifft ein Verfahren zur hochselektiven, abfallsäurefreien Herstellung von Mononitrotoluolen unter Ausnutzung der Reaktionswärme.

Mononitrotoluole sind wichtige Zwischenprodukte zur Herstellung von Kunststoffen, Farbstoffen und Hilfsmitteln. Mononitrotoluole werden technisch durch isotherme Nitrierung von Toluol bei niedrigen Temperaturen (20 bis 60°C) hergestellt. Dabei fallen große Mengen stark verunreinigter Abfallsäure an, die kostenintensiv entsorgt oder aufgearbeitet werden müssen. Nachteilig bei diesen Verfahren ist, daß eine erhebliche Reaktionswärme abgeführt werden muß und diese Energie auf einem niedrigen Temperaturniveau anfällt, so daß sie nicht verwertet werden kann.

Ein weiterer Nachteil besteht darin, daß zusätzliche Energie zur Aufkonzentrierung der kalten Gebrauchtsäure aufgewendet werden muß. Schwierigkeiten bereitet zusätzlich die Trennung von organischer und anorganischer Phase nach der Nitrierung. Restliches organisches Material muß aus der Gebrauchtsäure durch Extraktion mit Toluol entfernt werden. Ein bekanntes Phänomen ist dabei die Schwarzfärbung der Gebrauchtsäure ("black spent acid", US 4650912), die zu Problemen bei der Säureaufkonzentrierung führt.

Um den Anfall von Abfallsäure zu vermeiden, sind Verfahren anzustreben, die eine integrierte Schwefelsäureaufkonzentrierung unter Ausnutzung der Reaktionswärme beinhalten. Dies bedingt eine Kreislaufsäure, in der sich Nebenprodukte nicht anreichern dürfen und hohe Reaktionstemperaturen, um die Schwefelsäure technisch kostengünstig aufkonzentrieren zu können.

Für Benzol ist die adiabatische Mononitrierung in einer Reihe von Patentschriften beschrieben (US 2256999, US 4021498, US 4091042, US 4973770, EP 436443). Die oben genannten energetischen Nachteile bei der isothermen Toluol-Nitrierung entfallen bei der adiabatischen Benzol-Nitrierung, da deren Reaktionswärme auf höherem Temperatur-Niveau (Temperatur der Gebrauchtsäure am Ende der Reaktion beispielsweise > 100°C) anfällt und zur Aufkonzentrierung der Säure benutzt werden kann. Die Ausdehnung dieser Verfahrensweise auf die Mononitrierung von Toluol wird zwar in den oben genannten Anmeldungen erwähnt, ist aber nie in einem Beispiel beschrieben worden. Aus der Literatur ist hingegen bekannt, daß man Mononitrotoluole isotherm deshalb bei niedrigen Temperaturen (ca. 20-60°C) herstellt, um den bei höheren Temperaturen verstärkten Anfall von Dinitrotoluolen und Oxidationsprodukten, wie Nitrokresolen und Benzoesäuren, zu vermeiden (Houben-Weyl, Band X/1, S.156, Thieme Verlag, Stuttgart 1971; Kirk-Othmer, Vol.9, p.395, Interscience, New York 1952). Weiterhin ist bekannt, daß im Temperaturbereich von ca. 65-85°C bereits die isotherme Dinitrierung durchgeführt wird. Daher mußte angenommen werden, daß die adiabatische Nitrierung anderer Aromaten als Benzol nach den obigen Patentschriften rein spekulativen Charakter hat. Insbesondere schien die Temperaturangabe von 100°C für die Benzolnitrierung im Beispiel der US 4,973,770 nicht auf Toluol übertragbar zu sein.

Bei dem in US 4,973,770 beschriebenen Verfahren wird ferner auf eine einmalige Verdüsung und Feinverteilung des Benzols abgestellt, mit Hilfe derer der gesamte Reaktionsablauf bewältigt werden soll. Um die Feinverteilung möglichst lange aufrechtzuerhalten, muß die Koaleszenz der verdüsten Partikel untereinander und an der Wand vermieden werden. Zur Vermeidung der Wandberührung wird ein Reaktor mit großem Durchmesser, bezogen auf den Düsendurchmesser, eingesetzt; hierdurch wird eine starke Rückvermischung wie in einem ideal durchmischten Rührkessel bewirkt: Gemäß Ausführungsbeispiel von US 4.973.770 in Verbindung mit Fig. 1 wird Benzol durch eine Sprühdüse mit 0,5 mm Durchmesser in einen Reaktor mit einem Durchmesser von 75 mm eingeleitet. Bei einer Gesamtlänge des Reaktors von 430 mm wird die Mischsäure in einem Abstand von 150 mm von der Düse zugegeben, wo die Energie des Düsenstrahls bereits weitgehend für die Rückvermischung verbraucht ist. Die Vermischung der zugegebenen Mischsäure mit dem eingesprühten Benzol geschieht daher auf einem niedrigen Energieniveau und mit einer geringeren Intensität.

Auch der Anspruch von EP 436 443, demzufolge mit Nitronium-Ionen enthaltenden Mischsäuren mit molaren Zusammensetzungen gearbeitet wird, die der Fig. 6 in EP 436 443 zu entnehmen sind, ist außerhalb dessen, was dem Fachmann geläufig ist. So bezeichnet der in Fig. 6 herausgestellte Punkt D in Verbindung mit S.9, Z.10-12 von EP 436 443 eine Säure der Zusammensetzung von 72,02 Mol-% $H_2SO_4$, 2,99 Mol-% $HNO_3$ und 24,99 Mol-% $H_2O$, das entspricht 91,71 Gew.-% $H_2SO_4$, 2,45 Gew.-% $HNO_3$ und 5,84 Gew.-% $H_2O$. Eine derart starke Säure ist für die Toluolnitrierung, insbesondere für die Mononitrierung, schlecht geeignet. Darüberhinaus ist eine Kreisführung für die in diesem Verfahren entstehende Abfallsäure technisch nicht vertretbar, weil die notwendige Aufkonzentrierung auf weit über 90 Gew.-% zu aufwendig ist. Dieses Verfahren ist daher wirtschaftlich nicht sinnvoll und vermag dem Fachmann keinen Hinweis zur Lösung der vorliegenden Aufgabe zu vermitteln.

Wie überraschend gefunden wurde, läßt sich Toluol nach dem erfindungsgemäßen Verfahren mit hoher Selektivität adiabatisch bei höheren Reaktionstemperaturen mononitrieren, wenn die im folgenden beschriebenen Schritte und Bedingungen angewandt werden.

Die Erfindung betrifft ein Verfahren zur kontinuierlichen oder diskontinuierlichen Herstellung von Mononitrotoluolen durch Umsetzung von Toluol mit einem $HNO_3/H_2SO_4/H_2O$-Gemisch unter Bildung im wesentlichen der Mononitrotoluole und Reaktionswasser, gekennzeichnet durch die Schritte

a) Einspeisung der Reaktionsteilnehmer Toluol, $HNO_3$, $H_2SO_4$ und $H_2O$ in beliebiger Reihenfolge in einen mit Mischungsorganen ausgestatteten Reaktor, wobei

a1) die Menge an $HNO_3$ 1-8 Gew.-%, die Menge an $H_2SO_4$ 58-74 Gew.-% und die Menge an $H_2O$ den Rest zu 100 Gew.-% beträgt und 100 Gew.-% die Summe von $HNO_3$ + $H_2SO_4$ + $H_2O$ darstellen,

a2) das $H_2O$ als solches, als Verdünnungs-$H_2O$ der $HNO_3$, als Verdünnungs-$H_2O$ der $H_2SO_4$ oder in mehreren der genannten Formen eingesetzt wird und

a3) das molare Verhältnis von Toluol zu $HNO_3$ 0,9-1,5 beträgt,

b) rasche und intensive Vermischung der Gesamtmenge der Reaktionsteilnehmer, wozu eine Mischenergie von 1 bis 40 Watt pro Liter des gesamten Reaktionsgemisches, bevorzugt 3 bis 30 W/l, angewandt wird,

c) Durchführung der Umsetzung unter adiabatischen Bedingungen, wobei die Reaktionsteilnehmer mit solchen Temperaturen eingespeist werden, daß die Vermischung in Bereich von 20-110°C, bevorzugt 30-100°C, besonders bevorzugt 40-90°C erfolgt und die Temperatur am Ende der Reaktion 135°C nicht übersteigt,

d) Trennung des Reaktionsgemisches nach Durchführung der Reaktion in eine organische und eine anorganische Phase und

e) destillative Aufarbeitung der weitgehend $HNO_3$-freien anorganischen Phase unter Entfernung von Wasser.

Die Durchführung des erfindungsgemäßen Verfahrens erfolgt diskontinuierlich oder kontinuierlich, bevorzugt kontinuierlich.

Zur kontinuierlichen Durchführung kann beispielsweise so vorgegangen werden: Die Reaktionsteilnehmer werden in ihrer Gesamtmenge in einem Mischorgan rasch vermischt und als Mischung in einen Reaktor eingespeist. Die Mischungszeit bei kontinuierlicher Durchführung beträgt in der Regel weniger als 3 sek., beispielsweise 1 msek. bis 2,99 sek., bevorzugt 1 msek. bis 2 sek. Der Reaktor ist gegebenenfalls isoliert, verhindert die Rückvermischung weitgehend und wird adiabatisch betrieben. Zur weitgehenden Verhinderung der Rückvermischung ist der Reaktor unterteilt oder besteht aus mehreren Kammern oder Einheiten; an den Übergängen zwischen den Reaktorteilen wird das Reaktionsgemisch redispergiert. Das ausreagierte Gemisch läuft ab und wird in einem Trenngefäß getrennt; die Trennung erfolgt rasch. Die organische Phase wird wie üblich, z.B. durch Wäsche und Destillation aufgearbeitet oder sofort einer Zweitnitrierung zugeführt. Im allgemeinen, insbesondere bei einem Überschuß an Toluol, ist die abgetrennte anorganische Phase praktisch frei von Salpetersäure. Sollte dies, insbesondere bei einem Überschuß an Salpetersäure, nicht der Fall sein, kann restliche Salpetersäure in einem Nachreaktor unter Zusatz von weiterem Toluol im Sinne einer Reaktiv-Extraktion verbraucht werden. Die von der Salpetersäure weitgehend befreite anorganische Säurephase wird bevorzugt unter Ausnutzung der aufgenommenen Reaktionswärme und unter vermindertem Druck einer Flash-Verdampfung zugeführt. Hierbei wird Wasser aus der Säure entfernt und in bevorzugter Weise gleichzeitig die Säure auf die Eingangskonzentration und die Eingangstemperatur gebracht. Diese Säure ist dann als $H_2SO_4$ unmittelbar zum Einsatz in Schritt a) geeignet. Durch diese Rückführung der aufgearbeiteten anorganischen Phase ($H_2SO_4$, $H_2O$) in den Prozeß ergibt sich eine Kreislauffahrweise für die $H_2SO_4$; es kann sinnvoll sein, einen geringen Teil dieser $H_2SO_4$ auszuschleusen, um etwaige Verunreinigung auf einem niedrigen Niveau zu lassen. Für den Fall, daß die anorganische Phase noch Toluol, Nitrotoluol und evtl. organische Nebenprodukte enthält, kann es sinnvoll sein, die anorganische Phase vor der Flash-Verdampfung zur Entfernung der Organika zu strippen. Das danach als Flash-Kondensat erhaltene Wasser ist dann von höherer Reinheit, und seine Entsorgung ist einfacher. Selbstverständlich kann auch das Flash-Kondensat, z.B. durch Strippen oder Phasentrennung von Organika befreit werden, wobei analog ein restliches Flash-Kondensat bzw. eine Wasser-Säure-Phase mit höherer Reinheit zurückbleibt. Die bei der Nachreaktion der $HNO_3$ mit weiterem Toluol sowie beim Strippen oder sonstigen Stofftrennungen, wie Phasentrennung, anfallenden Organika können dem Prozeß an geeigneter Stelle zugesetzt werden (Toluol, (Di)Nitrotoluol) oder werden ausgeschleust und entsorgt (Verunreinigungen, Nebenprodukte).

Die Reaktionsteilnehmer können gemeinsam, jedoch auch einzeln oder als Gemische zweier oder dreier von ihnen gleichzeitig oder sukzessive dem mit Mischungsorganen ausgestatteten Reaktor zugeführt werden. Die Vermischung der Einsatzstoffe kann beispielsweise so stattfinden, daß man Toluol und Salpetersäure oder gegebenenfalls Wasser als separate Ströme gleichzeitig oder sukzessive der aufkonzentrierten, recyclisierten Schwefelsäure zusetzt, wobei die Salpetersäure durch Wasser und/oder Schwefelsäure und Wasser verdünnt sein kann. Toluol kann auch mit Wasser und Schwefelsäure vorvermischt werden, und die resultierende Emulsion wird mit Salpetersäure, die mit Schwefelsäure und/oder Wasser vermischt sein kann, weiter intensiv gemischt. Weiterhin kann auch das Toluol mit einer Mischsäure aus Schwefelsäure, Salpetersäure und Wasser intensiv vermischt und dann erfindungsgemäß weiter behandelt werden. Noch weitere Varianten der Zuführung der Reaktionsteilnehmer, ihrer intensiven Vermischung und Weiterbehandlung sind für den Fachmann leicht erkennbar. Dazu sind die in der Technik bekannten Mischorgane geeignet, z.B.: 1. Statische Mischer, 2. Pumpen, 3. Düsen, 4. Rührer oder Kombinationen hiervon.

Für das Gelingen der Reaktion ist es von geringerer Bedeutung, in welcher Reihenfolge und Zusammensetzung die Reaktionsteilnehmer Salpetersäure und Toluol sowie Schwefelsäure und Wasser miteinander vermischt werden, solange das Reaktionsgemisch nach der Gesamtvermischung die erfindungsgemäße Zusammensetzung besitzt und die Vermischung in der erfindungsgemäßen Intensität und bei kontinuierlich durchgeführter Reaktion weitgehend rückvermischungsfrei stattfindet.

Die Intensität der Vermischung kann bei der diskontinierlichen Fahrweise neben dem hohen Energieeintrag auch durch die kurze Zugabezeit der Reaktionsteilnehmer gekennzeichnet werden, die 0,001 bis 15 %, bevorzugt 0,001 bis 3 % der Zeit beträgt, die für den Ablauf der Reaktion zwischen Toluol und Salpetersäure erforderlich ist. Damit ist die Durchführung des erfindungsgemäßen Verfahrens auch absatzweise in einem Rührkessel möglich.

Der Einspeisung und intensiven Vermischung der Reaktionsteilnehmer folgen bei kontinuierlicher Durchführung mindestens zwei Redispergierungen. Hierzu sind im Reaktor vorzugsweise abschnittweise statische Mischelemente, gegebenenfalls auch in Form von sphärisch geformten festen Einbauten, wie Lochbleche, Schlitzbleche, Prallbleche, Strömungsbrecher oder Rührer oder ähnliche, dem Fachmann für diesen Zweck bekannte Einbauten bzw. Organe vorhanden.

Als kontinuierlich betriebene Reaktoren für das erfindungsgemäße Verfahren seien beispielsweise genannt: Rohrreaktoren mit Einbauten zur Redispergierung, wie Strömungsbrechern, Umlenkblechen, Statikmischern oder Rührern und ähnlichen; stark gerührte Kessel in Kaskadenanordnung; Schlaufenreaktoren mit Einbauten wie oben; Kombinationen mehrerer der genannten Apparate; weitere gleichwirkende Reaktoren, wie Kammerreaktoren mit Rührern in jeder Kammer. In bevorzugter Weise werden Rohrreaktoren mit Einbauten eingesetzt. Die Einbauten sind bevorzugt, gegebenenfalls sphärisch geformte Lochbleche. Alle Einbauten stellen Unterteilungen der Gesamtapparatur dar, die gleichermaßen der Redispergierung und der weitgehenden Verhinderung der Rückvermischung dienen.

Nach dem intensiven Vermischen, nach jeder Dispergierung bzw. nach dem Durchströmen einer gewissen Teillänge des Reaktors wird ein Koaleszieren der Dispersionströpfchen beobachtet, was durch Redispergierung rückgängig gemacht werden kann. Die Zahl der Redispergierungsvorgänge beträgt erfindungsgemäß 2 bis 50, bevorzugt 3 bis 30, besonders bevorzugt 4 bis 20. Zur Überwindung der dabei auftretenden Druckverluste wird mit den Reaktionsteilnehmern eine Mischenergie pro Liter des gesamten Reaktionsgemisches von 1 bis 40 Watt/Liter, bevorzugt 3 bis 30 W/l, in das Reaktionssystem gegeben.

Die Vermischung der Reaktionsteilnehmer erfolgt im Bereich von 20 bis 110°C, bevorzugt 30 bis 100 °C, besonders bevorzugt 40 bis 90 °C. Es werden adiabatische Reaktionsbedingungen eingehalten. Die Endtemperatur ist abhängig von der Höhe der Mischungstemperatur, von den Mengenverhältnissen der Reaktionsteilnehmer und vom Umsatz; sie übersteigt im allgemeinen 135°C nicht, meistens nicht 125°C.

Der Gehalt an zugesetzter Salpetersäure im Reaktionsgemisch zum Zeitpunkt der Vermischung beträgt, bezogen auf die Summe von Salpetersäure, Schwefelsäure und Wasser, 1 bis 8 Gew.-%, bevorzugt 1 bis 6 Gew.-%, besonders bevorzugt 1,5 bis 4 Gew.-%. Salpetersäure kann in hochkonzentrierter oder in azeotrop siedender Form, bevorzugt aber in Form der ca. 60-65 Gew.-% aufweisenden und billig verfügbaren "Schwachsäure" eingesetzt werden.

Der Gehalt an Schwefelsäure im Reaktionsgemisch zum Zeitpunkt der Vermischung beträgt, bezogen auf die Summe von Salpetersäure, Schwefelsäure und Wasser, 58-74 Gew.-%, bevorzugt 60-72 Gew.-%, besonders bevorzugt 61-69 Gew.-%. In diesen Zahlen sind die bei einer Kreislauffahrweise der $H_2SO_4$ gegebenenfalls enthaltenen prozessspezifischen Verunreinigungen nicht eingerechnet.

Der Rest zu 100 Gew.-% ist $H_2O$. Dies kann als solches, als Verdünnungs-$H_2O$ der $H_2SO_4$, als Verdünnungs-$H_2O$ der $HNO_3$ oder in mehreren der genannten Formen eingesetzt werden. In bevorzugter Form liegt $H_2O$ als Verdünnungs-$H_2O$ sowohl der $H_2SO_4$ als auch der $HNO_3$ vor.

Da die Nitrierstärke bei wechselnden Salpetersäuregehalten in der Nitriersäure abhängig ist vom Verhältnis von Schwefelsäure zu Wasser, wird sie anhand der Schwefelsäurekonzentration der ablaufenden und weitgehend salpetersäurefreien Gebrauchtsäure bestimmt und gegebenenfalls nachreguliert. Diese $H_2SO_4$-Konzentration der Gebrauchtsäure soll erfindungsgemäß 62 bis 74 Gew.-%, bevorzugt 64 bis 72 Gew.-%, besonders bevorzugt 66 bis 70 Gew.-% betragen. Zum Wiedereinsatz wird die ablaufende Schwefelsäure um 0,6 - 7 Prozentpunkte, in vielen Fällen um 1,5 - 3 Prozentpunkte aufkonzentriert, wobei Wasser (Reaktionswasser, gegebenenfalls Verdünnungswasser) destillativ ausgeschleust wird. Hierzu wird in bevorzugter Weise die von der ablaufenden $H_2SO_4$ infolge der adiabatischen Reaktionsführung aufgenommene Reaktionswärme ausgenutzt und unter vermindertem Druck im Bereich von 1 bis 100 mbar, vorzugsweise bei 5 - 80 mbar, besonders bevorzugt 10-75 mbar, gearbeitet. Beispielsweise kann dies in Form einer Flash-Verdampfung durchgeführt werden. Die dabei gewonnene $H_2SO_4$ ist zum Einsatz in Schritt a) geeignet. In bevorzugter Weise wird die destillative Ausschleusung von Wasser so durchgeführt, daß die Temperatur und Konzentration der aufkonzentrierten $H_2SO_4$ unmittelbar den in Schritt a) geforderten Werten entspricht. Eine solche Nutzung der Reaktionswärme macht das erfindungsgemäße Verfahren wirtschaftlicher als die bekannten Verfahren zur Herstellung von Nitrotoluolen.

Mögliche Ausführungsformen bezüglich der Mischsäuren mit wechselnden Zusammensetzungen, ablaufender $H_2SO_4$-Konzentrationen, Temperaturführung und Druck der Flash-Verdampfung sowie Aufkonzentration der $H_2SO_4$ seien beispielhaft wie folgt zusammengestellt (Fälle a, b und c):

| | a | b | c |
|---|---|---|---|
| Mischsäure | | | |
| $HNO_3$ (Gew.-%) | 4,00 | 3,00 | 2,50 |
| $H_2SO_4$ (Gew.-%) | 64,11 | 65,56 | 66,79 |
| $H_2O$ (Gew.-%) | 31,89 | 31,44 | 30,71 |
| Stärke der eingesetzten Säuren | | | |
| $HNO_3$ (Gew.-%) | 60,0 | 60,0 | 60,0 |
| $H_2SO_4$ (Gew.-%) | 68,69 | 69,01 | 69,69 |
| ablaufende $H_2SO_4$ (Gew.-%) | 66,0 | 67,0 | 68,0 |
| Mischungstemperatur (°C) | 80 | 85 | 90 |
| Endtemperatur (°C), ca. | 120 | 115 | 115 |
| Druck bei Flash-Verdampfung (ca. mbar) | 40 | 50 | 60 |

Das molare Verhältnis von Toluol zu $HNO_3$ beträgt allgemein 0,9-1,5. Um die Bildung unerwünschter Dinitrototuole zu minimieren, beträgt das Molverhältnis von Toluol zu Salpetersäure bevorzugt 1,0 bis 1,5, besonders bevorzugt 1,03 bis 1,3, ganz besonders bevorzugt 1,05 bis 1,2. Für den Fall, daß die erfindungsgemäß erhältlichen Nitrotoluole der Dinitrierung zugeführt werden sollen, sind aber auch weitere molare Bereiche, z.B. 0,9 - 1,2 Mol, bevorzugt 0,9 - 1,05 Mol, besonders bevorzugt 0,95 - 1 Mol Toluol pro Mol Salpetersäure zulässig.

Die Reaktion des erfindungsgemäßen Verfahrens lautet formelmäßig:

$$C_6H_5-CH_3 + HNO_3 \rightarrow O_2N\text{-}C_6H_4-CH_3 + H_2O$$

Somit werden Toluol und $HNO_3$ in das Verfahren eingeführt und Mononitrotoluol und $H_2O$ ausgeschleust, während das beschriebene $H_2SO_4/H_2O$-Gemisch das Reaktionsmedium darstellt. Da bei der technischen Realisierung vorteilhafterweise, in Abhängigkeit von den Preisen der jeweils verfügbaren Salpetersäuren, verdünnte Salpetersäuren eingesetzt werden, muß zusätzlich zum Reaktionswasser auch noch Verdünnungs-$H_2O$ der $HNO_3$ ausgeschleust werden.

Die bei der Trennung des Reaktionsgemisches anfallende organische Phase kann auf reines Mononitrotoluol aufgearbeitet werden oder der Dinitrotoluol-Herstellung zugeführt werden. Im ersteren Fall wird man, wie oben beschrieben, mindestens molare Mengen an Toluol oder einen geringen molaren Überschuß einsetzen, um sowohl die $HNO_3$ zu verbrauchen als auch die Zweitnitrierung zu unterdrücken; ein etwaiger Toluolüberschuß wird aus der abgetrennten organischen Phase abdestilliert. Zuvor kann die organische Phase gewaschen werden, um wasser-, säure- oder alkalilösliche Verunreinigungen abzutrennen, wie anorganische und organische Säuren und phenolische Verunreinigungen. Die Bildung von Oxidationsprodukten (Phenolkörper, Oxidation der $CH_3$-Gruppe) ist im erfindungsgemäßen Verfahren jedoch weit zurückgedrängt. Ebenso ist die Bildung von Dinitrotoluolen stark unterdrückt. Diese Dinitrotoluole stören jedoch dann nicht, wenn ohnehin eine Zweitnitrierung vorgesehen ist; daher darf in solchen Fällen auch mit einem Toluolunterschuß gearbeitet werden.

Als Modell für einen kontinuierlich betriebenen rückvermischungsfreien technischen Reaktor und zur Darstellung der diskontinuierlichen Durchführung kann im Labor ein Batch-Ansatz in einem stark gerührten, wärmeisolierten Rührkolben, z.B. in einem sogenannten Sulfierbecher, der mit Stromstörern versehen ist, dienen. Dabei werden Toluol, Schwefelsäure und Wasser z.B. bei 85°C vorgelegt und mit der auf die erfindungsgemäße Einsatztemperatur erwärmten Salpetersäure, die durch Wasser und/oder Schwefelsäure verdünnt sein kann, in ca. 1 bis 2 Sekunden versetzt. Alternativ zur beschriebenen Dosiervariante kann beispielsweise die Gesamtmenge an Salpetersäure, Schwefelsäure und Wasser bei der erfindungsgemäßen Einsatztemperatur vorgelegt und mit auf die erfindungsgemäße Einsatztemperatur erwärmtem Toluol in ca. 1 bis 2 Sekunden versetzt werden. Nach der Dosierung läßt man die Reaktion in beiden Fällen unter starkem Rühren adiabatisch ablaufen. Noch weitere Dosiervarianten sind für den Fachmann leicht erkennbar. Die Endtemperatur, gleichbedeutend mit dem Ende der Reaktion, wird je nach Energieeintrag über den Rührer und je nach Konzentration der $H_2SO_4$ innerhalb von maximal 300 Sekunden erreicht. Der Inhalt des Reaktionsgefäßes entspricht dabei im zeitlichen Verlauf einem Volumenteil in der axialen Bewegung durch einen Rohrreaktor mit Pfropfenströmung. Was im Batch-Ansatz zeitlich nacheinander geschieht, läuft etwa in einem Rohrreaktor örtlich hintereinander ab.

Bei dieser Ausführung des erfindungsgemäßen Verfahrens im Labor werden nach der Aufarbeitung im Rahmen der analytischen Schwankungsbreiten Mononitrotoluole etwa mit folgender Isomerenverteilung erhalten:

| 2-Nitrotoluol | ca. 58-60 % |
| 3-Nitrotoluol | ca. 4-6 % |
| 4-Nitrotoluol | ca. 35-37 % |

Die Ausbeute an Mononitrotoluolen, bezogen auf eingesetzte Salpetersäure, beträgt hierbei > 95 % der theoretischen Ausbeute, vielfach > 97 %; sie erreicht bei kontinuierlicher Durchführung Werte > 98 %.

Ist ein Reaktionsgemisch mit einem relativ niedrigen Gehalt an 3-Nitrotoluol erwünscht, so wird vorzugsweise die Vermischung der Reaktionsteilnehmer im unteren Temperaturbereich von etwa 20 bis 80°C vorgenommen und gegebenenfalls zusätzlich mit relativ niedrigen Konzentrationen an zugesetzten $HNO_3$ von 1 bis 2,5 Gew.-% gearbeitet. In diesem Fall fällt die weitgehend $HNO_3$-freie Abfallsäure ebenfalls bei niedrigerer Temperatur an, so daß eine Flash-Aufkonzentrierung bei niedrigeren Drücken, z.B. bei 1-70 mbar durchgeführt wird.

Nach Erreichen der Reaktionsendtemperatur wird der Rührer angehalten. Die Phasen trennen sich in ca. 20 Sekunden. Die Dimensionierung eines kontinuierlichen technischen Reaktors wird bevorzugt so bemessen, daß das Reaktionsgemisch die Reaktionsendtemperatur im Reaktor erreicht.

Die nach der Reaktion auf dem Niveau der jeweiligen Reaktionsendtemperatur abgetrennte Säurephase ist von hellgelber bis hellbrauner Farbe und wird in der oben beschriebenen Weise aufkonzentriert, wobei die oben beschriebene Reaktivextraktion eingeschoben werden kann. Die so geführte Kreislauf-$H_2SO_4$ enthält dann weniger als 25 ppm Salpetersäure und weniger als 25 ppm salpetrige Säure, z.B. je 5 bis 20 ppm sowie geringe Mengen an organischen, kohlenstoffhaltigen Verunreinigungen.

Wird von der erfindungsgemäßen Durchführung der Reaktion abgewichen, z.B. in der Art, daß man etwa bei der Durchführung im Labormaßstab Toluol, Schwefelsäure und Wasser bei der erfindungsgemäßen Einsatztemperatur in einem wärmeisolierten Rührkolben (Sulfierbecher) vorlegt und mit Salpetersäure, die durch Wasser und Schwefelsäure verdünnt ist, langsam z.B. in 30 Minuten versetzt, dabei von der intensiven und raschen Durchmischung der Gesamtmenge aller Reaktionsteilnehmer abweicht und somit eine weitgehend vollständige, jedoch erfindungsgemäß unerwünschte Rückvermischung zuläßt, wird ein schwarzes Reaktionsgemisch erhalten (Beispiel 3). Die teerige organische Phase enthält in ihren flüchtigen Anteilen noch ca. 40 bis 50 % an nicht umgesetztem Toluol. Da entsprechende, in einem Reaktionskalorimeter durchgeführte Versuche zeigten, daß diese Reaktionsführung mit einer gegenüber der erfindungsgemäßen Verfahrensweise ca. doppelten Reaktionsenthalpie verläuft und mit einer starken Gasentwicklung verbunden ist, wurde von weiteren Untersuchungen und Aufklärungen in dieser Richtung abgesehen.

**Beispiel 1**

In einem wärmeisolierten Sulfierbecher (Durchmesser 100 mm), versehen mit Stromstörern und 2 auf einer Welle sitzenden Turbinenrührern (Durchmesser 39,9 mm) wurden bei 85°C 653,8 g Schwefelsäure und 297,9 g Wasser vorgelegt und mit 1800 U/min gerührt. Die dadurch eingebrachte spezifische Rührleistung betrug 22 W/l. In 1 bis 2 Sekunden gab man dann 50,7 g (0,55 mol) Toluol und unmittelbar danach in ebenfalls 1 bis 2 Sekunden eine auf 85°C erwärmte Mischung von 31,5 g (0,5 mol) Salpetersäure, 34,4 g Schwefelsäure und 32,6 g Wasser zu und ließ ohne Kühlung reagieren. Nach 105 Sekunden war die Endtemperatur von 110,5°C erreicht und man hielt den Rührer an. Die Phasentrennung war nach 15 Sekunden vollständig. Man isolierte 71,7 g organische Phase folgender Zusammensetzung (geeichte GC):

| Toluol | 6,00 % |
| 2-Nitrotoluol | 54,20 % (rel. 58,3 %) |
| 3-Nitrotoluol | 5,60 % (rel. 6,02 %) |
| 4-Nitrotoluol | 33,20 % (rel. 35,7 %) |
| 2,4-Dinitrotoluol | 0,14 % |
| 2,6-Dinitrotoluol | 0,05 % |
| Dinitro-p-kresol | 0,60 % |

(fortgesetzt)

| | |
|---|---|
| Dinitro-o-kresol | 0,17 % |
| Unbekannte | Rest |

Dies entspricht einer Ausbeute an Mononitrotoluolen von 97,2 % der theoretischen Ausbeute. Die in der anorganischen Phase enthaltenen Anteile von bis zu 2 % sind durch Extraktion oder Destillation zugänglich.

**Beispiel 2**

In einem wärmeisolierten Sulfierbecher wie in Beispiel 1 wurden bei 85°C 688,5 g Schwefelsäure, 330,2 g Wasser und 31,5 g (0,5 mol) Salpetersäure vorgelegt und mit 1200 U/min gerührt. Die dadurch eingebrachte spezifische Rührleistung betrug 8,7 W/l. In 1 bis 2 Sekunden gab man 55,3 g (0,6 mol) auf 85°C erwärmtes Toluol zu und ließ ohne Kühlung reagieren. Nach 110 Sekunden war die Endtemperatur von 110,5°C erreicht und man hielt den Rührer an. Die Phasentrennung war nach 20 Sekunden vollständig. Man isolierte 73,8 g organische Phase folgender Zusammensetzung (geeichte GC):

| | |
|---|---|
| Toluol | 10,50 % |
| 2-Nitrotoluol | 51,40 % (rel. 58,1 %) |
| 3-Nitrotoluol | 5,40 % (rel. 6,10 %) |
| 4-Nitrotoluol | 31,70 % (rel. 35,8 %) |
| 2,4-Dinitrotoluol | 0,13 % |
| 2,6-Dintrotoluol | 0,05 % |
| Dinitro-p-kresol | 0,51 % |
| Dinitro-o-kresol | 0,18 % |
| Unbekannte | Rest |

Dies entspricht einer Ausbeute an Mononitrotoluolen in der organischen Phase von 95,2 % der theoretischen Ausbeute.

**Beispiel 3** (zum Vergleich)

In einem wärmeisolierten Sulfierbecher wie in Beispiel 1 wurden bei 85°C 653,8 g Schwefelsäure, 297,9 g Wasser und 50,7 g (0,55 mol) Toluol vorgelegt und mit 1800 U/min gerührt (22 W/l spezifische Rührleistung). In 30 Minuten dosierte man eine auf 85°C erwärmte Mischung von 31,5 g (0,5 mol) Salpetersäure, 34,4 g Schwefelsäure und 32,6 g Wasser zu und ließ ohne Kühlung reagieren. Die Temperatur stieg dabei auf 100,5°C (Wärmeverluste durch die lange Reaktionszeit), und das gesamte Reaktionsgemisch färbte sich unter Schäumen schwarz. Nach Phasentrennung (mehr als 16 Stunden) wurden 53,0 g schwarze organische Phase isoliert, deren flüchtige Anteile die folgende Zusammensetzung besitzen:

| | |
|---|---|
| Toluol | 41,9 % |
| 2-Nitrotoluol | 31,1 % |
| 3-Nitrotoluol | 3,1 % |
| 4-Nitrotoluol | 18,6 % |
| Unbekannte | Rest |

**Beispiel 4** (zum Vergleich)

In einem wärmeisolierten Sulfierbecher wie in Beispiel 1 wurden bei 85°C 1133,6 g Schwefelsäure und 55,9 g Wasser vorgelegt und mit 1800 U/min gerührt (22 W/l spezifische Rührleistung). In ca. 1 bis 2 Sekunden gab man dann 50,7 g (0,55 mol) Toluol und unmittelbar danach in ebenfalls 1 bis 2 Sekunden eine auf 85°C erwärmte Mischung von 31,5 g (0,5 mol) Salpetersäure, 47,7 g Schwefelsäure und 19,3 g Wasser zu und ließ ohne Kühlung reagieren. Nach 20 Sekunden war die Endtemperatur von 120°C erreicht und man hielt den Rührer an. Die homogene, braune Lösung wurde auf ca. 1 kg Eis abgelassen und der dabei abgeschiedene Feststoff abgesaugt. Der feuchte Feststoff (48,4 g) enthielt laut HPLC 32,1 Gew.-% 4-Nitrotoluol-2-sulfonsäure (Rest: Wasser und Schwefelsäure).

Aus der Mutterlauge wurden durch Extraktion mit Methylenchlorid 1,9 g Organika folgender Zusammensetzung

isoliert:

| | |
|---|---|
| 2-Nitrotoluol: | 3,7 % |
| 3-Nitrotoluol: | 0,4 % |
| 4-Nitrotoluol: | 0,1 % |
| 2,4-Dinitrotoluol: | 78,2 % |
| 2,6-Dinitrotoluol: | 6,1 % |
| Unbekannte: | Rest |

Die extrahierte wässrige Phase (2250 g) enthielt laut HPLC 3,3 Gew.-% 2-Nitrotoluol-4-sulfonsäure.

**Beispiel 5**

In einem wärmeisolierten Sulfierbecher wie in Beispiel 1 wurden bei 50°C 662,2 g Schwefelsäure, 313,8 g Wasser und 25,3 g (0,275 mol) Toluol vorgelegt und mit 1800 U/min gerührt (22 W/l spezifische Rührleistung). In 1 - 2 Sekunden gab man eine auf 50°C erwärmte Mischung von 15,8 g (0,25 mol) Salpetersäure, 33,9 g Schwefelsäure und 24,6 g Wasser zu und ließ ohne Kühlung reagieren. Nach 220 Sekunden war die Endtemperatur von 64°C erreicht und man hielt den Rührer an. Die Phasentrennung war nach 90 Sekunden vollständig. Man isolierte 35,5 g organische Phase folgender Zusammensetzung (geeichte GC):

| | |
|---|---|
| Toluol | 6,2 % |
| 2-Nitrotoluol | 55,3 % |
| 3-Nitrotoluol | 4,61 % |
| 4-Nitrotoluol | 31,9 % |
| 2,4-Dinitrotoluol | 0,01 % |
| 2,6-Dinitrotoluol | 0,0 % |
| Dinitro-p-kresol | 0,86 % |
| Dinitro-o-kresol | 0,22 % |
| Unbekannte | Rest |

Dies entspricht einer Ausbeute an Mononitrotoluolen von 95,0 % der theoretischen Ausbeute.

**Beispiel 6**

In einem wärmeisolierten Sulfierbecher wie in Beispiel 1 wurden bei 70°C 662,2 g Schwefelsäure, 313,8 g Wasser und 25,3 g (0,275 mol) Toluol vorgelegt und mit 1800 U/min gerührt (22 W/l spezifische Rührleistung). In 1 - 2 Sekunden gab man eine auf 70°C erwärmte Mischung von 15,8 g (0,25 mol) Salpetersäure, 33,9 g Schwefelsäure und 24,6 g Wasser zu und ließ ohne Kühlung reagieren. Nach 180 Sekunden war die Endtemperatur von 84°C erreicht und man hielt den Rührer an. Die Phasentrennung war nach 50 Sekunden vollständig. Man isolierte 35,6 g organische Phase folgender Zusammensetzung (geeichte GC):

| | |
|---|---|
| Toluol | 6,3 % |
| 2-Nitrotoluol | 54,4 % |
| 3-Nitrotoluol | 5,05 % |
| 4-Nitrotoluol | 32,6 % |
| 2,4-Dinitrotoluol | 0,02 % |

(fortgesetzt)

| | |
|---|---|
| 2,6-Dinitrotoluol | 0,01 % |
| Dinitro-p-kresol | 0,71 % |
| Dinitro-o-kresol | 0,22 % |
| Unbekannte | Rest |

Dies entspricht einer Ausbeute an Mononitrotoluolen von 95,6 % der theoretischen Ausbeute.

**Beispiel 7**

In einem wärmeisolierten Sulfierbecher wie in Beispiel 1 wurden bei 50°C 339,2 g Schwefelsäure, 128,9 g Wasser und 33,7 g (0,366 mol) Toluol vorgelegt und mit 1800 U/min gerührt (22 W/l spezifische Rührleistung). In 1 - 2 Sekunden gab man eine auf 50°C erwärmte Mischung von 21,0 g (0,333 mol) Salpetersäure, 17,9 g Schwefelsäure und 6,8 g Wasser zu und ließ ohne Kühlung reagieren. Nach 165 Sekunden war die Endtemperatur von 85°C erreicht und man hielt den Rührer an. Die Phasentrennung war nach 50 Sekunden vollständig. Man isolierte 47,5 g organische Phase folgender Zusammensetzung (geeichte GC):

| | |
|---|---|
| Toluol | 6,2 % |
| 2-Nitrotoluol | 54,6 % |
| 3-Nitrotoluol | 4,7 % |
| 4-Nitrotoluol | 33,6 % |
| 2,4-Dinitrotoluol | 0,11 % |
| 2,6-Dinitrotoluol | 0,06 % |
| Dinitro-p-kresol | 0,49 % |
| Dinitro-o-kresol | 0,07 % |
| Unbekannte | Rest |

Dies entspricht einer Ausbeute an Mononitrotoluolen von 96,7 % der theoretischen Ausbeute.

**Beispiel 8**

In einem wärmeisolierten Sulfierbecher wie in Beispiel 1 wurden bei 50 °C 653,8 g Schwefelsäure, 297,9 g Wasser und 50,7 g (0,55 mol) Toluol vorgelegt und mit 1800 U/min gerührt (22 W/l spezifische Rührleistung). In 1 - 2 Sekunden gab man eine auf 50 °C erwärmte Mischung von 31,5 g (0,5 mol) Salpetersäure, 34,4 g Schwefelsäure und 32,6 g Wasser zu und ließ ohne Kühlung reagieren. Nach 203 Sekunden war die Endtemperatur von 75 °C erreicht und man hielt den Rührer an. Die Phasentrennung war nach 45 Sekunden vollständig. Man isolierte 72,4 g organische Phase folgender Zusammensetzung (geeichte GC):

| | |
|---|---|
| Toluol: | 6,50 % |
| 2-Nitrotoluol: | 55,10 % |
| 3-Nitrotoluol: | 4,70 % |
| 4-Nitrotoluol: | 32,1 % |
| 2,4-Dinitrotoluol: | 0,02 % |

| 2,6-Dinitrotoluol: | 0,01 % |
|---|---|
| Dinitro-p-kresol: | 0,69 % |
| Dinitro-o-kresol: | 0,16 % |
| Unbekannte: | Rest |

Dies entspricht einer Ausbeute an Mononitrotoluolen von 97,0 % der theoretischen Ausbeute.

**Beispiel 9**

In einem wärmeisolierten Sulfierbecher wie in Beispiel 1 wurden bei 25°C 450,8 g Schwefelsäure, 158,9 g Wasser und 46,1 g (0,5 mol) Toluol vorgelegt und mit 1200 U/min gerührt (8,7 W/l spezifische Rührleistung). In 1 - 2 Sekunden gab man eine auf 25°C erwärmte Mischung von 21,0 g (0,333 mol) Salpetersäure, 23,7 g Schwefelsäure und 19,7 g Wasser zu und ließ ohne Kühlung reagieren. Nach 315 Sekunden war die Endtemperatur von 52 °C erreicht und man hielt den Rührer an. Die Phasentrennung war nach 75 Sekunden vollständig. Man isolierte 60,4 g organische Phase folgender Zusammensetzung (geeichte GC):

| Toluol | 25,60 % |
|---|---|
| 2-Nitrotoluol | 42,86 % |
| 3-Nitrotoluol | 3,14 % |
| 4-Nitrotoluol | 26,41 % |
| 2,4-Dinitrotoluol | 0,02 % |
| 2,6-Dinitrotoluol | 0,01 % |
| Dinitro-p-kresol | 0,31 % |
| Dinitro-o-kresol | 0,08 % |
| Unbekannte | Rest |

Dies entspricht einer Ausbeute an Mononitrotoluolen von 95,8 % der theoretischen Ausbeute.

**Patentansprüche**

1. Verfahren zur kontinuierlichen oder diskontinuierlichen Herstellung von Mononitrotoluen durch Umsetzung von Toluol mit einem $HNO_3/H_2SO_4/H_2O$-Gemisch unter Bildung im wesentlichen der Mononitrotoluole und Reaktionswasser, gekennzeichnet durch die Schritte

    a) Einspeisung der Reaktionsteilnehmer Toluol, $HNO_3$, $H_2SO_4$ und $H_2O$ in beliebiger Reihenfolge in einen mit Mischungsorganen ausgestatteten Reaktor, wobei

    a1) die Menge an $HNO_3$ 1-8 Gew.-%, die Menge an $H_2SO_4$ 58 - 74 Gew.-% und die Menge an $H_2O$ den Rest zu 100 Gew.-% beträgt und 100 Gew.-% die Summe von $HNO_3 + H_2SO_4 + H_2O$ darstellen,

    a2) das $H_2O$ als solches, als Verdünnungs-$H_2O$ der $HNO_3$, als Verdünnungs-$H_2O$ der $H_2SO_4$ oder in mehreren der genannten Formen eingesetzt wird und

    a3) das molare Verhältnis von Toluol zu $HNO_3$ 0,9 - 1,5 beträgt,

    b) rasche und intensive Vermischung der Gesamtmenge der Reaktionsteilnehmer, wozu eine Mischenergie von 1 bis 40 Watt pro Liter des gesamten Reaktionsgemisches, bevorzugt 3 bis 30 W/l, angewandt wird,

    c) Durchführung der Umsetzung unter adiabatischen Bedingungen, wobei die Reaktionsteilnehmer mit solchen Temperaturen eingespeist werden, daß die Vermischung im Bereich von 20-110°C, bevorzugt 30-100°C, besonders bevorzugt 40-90°C, erfolgt und am Ende der Reaktion 135°C nicht übersteigt,

d) Trennung des Reaktionsgemisches nach Durchführung der Reaktion in eine organische und eine anorganische Phase und

e) destillative Aufarbeitung der weitgehend HNO$_3$-freien anorganischen Phase unter Entfernung von Wasser.

2. Verfahren nach Anspruch 1, dadurch gekennzeichnet, daß es kontinuierlich durchgeführt wird und hierzu Rohrreaktoren eingesetzt werden, die die Rückvermischung der Reaktionsteilnehmer weitgehend verhindern.

3. Verfahren nach Anspruch 2, dadurch gekennzeichnet, daß die Reaktionsteilnehmer vor Eintritt in den die Rückvermischung weitgehend verhindernden Reaktor mit Hilfe von Mischorganen innerhalb einer Zeit von weniger als 3 sek. intensiv vermischt werden und beim Durchströmen des Reaktors mindestens 2mal redispergiert werden.

4. Verfahren gemäß Anspruch 1, dadurch gekennzeichnet, daß im Schritt e) die infolge der adiabatischen Reaktionsführung von der anorganischen Phase aufgenommene Reaktionswärme zur destillativen Ausschleusung von Wasser unter einem Druck von 1-100 mbar, bevorzugt 5-80 mbar, besonders bevorzugt 10-75 mbar ausgenutzt wird und bevorzugt dabei eine H$_2$SO$_4$ erzeugt wird, die zum Einsatz in Schritt a) geeignet ist.

5. Verfahren gemäß Anspruch 1, dadurch gekennzeichnet, daß der Schwefelsäuregehalt in der Säurephase nach dem Schritt d) von 62 bis 74 Gew.-%, bevorzugt 64 bis 72 Gew.-%, besonders bevorzugt 66 bis 70 Gew.-% beträgt.

6. Verfahren gemäß Anspruch 1, dadurch gekennzeichnet, daß der Gehalt an zugesetzter Salpetersäure im Reaktionsgemisch zum Zeitpunkt der Vermischung, bezogen auf die Summe von Salpetersäure, Schwefelsäure und Wasser, 1 bis 6 Gew.-%, bevorzugt 1,5 bis 4 Gew.-% beträgt.

7. Verfahren gemäß Anspruch 1, dadurch gekennzeichnet, daß der Gehalt an Schwefelsäure im Reaktionsgemisch zum Zeitpunkt der Vermischung, bezogen auf die Summe von Salpetersäure, Schwefelsäure und Wasser, 60-72 Gew.-%, bevorzugt 61-69 Gew.-% beträgt.

8. Verfahren gemäß Anspruch 1, dadurch gekennzeichnet, daß das Molverhältnis von Toluol zu Salpetersäure größer 1,0 bis 1,5, bevorzugt 1,03 bis 1,3, besonders bevorzugt 1,05 bis 1,2 beträgt.

9. Verfahren nach Anspruch 1, dadurch gekennzeichnet, daß bei der destillativen Aufarbeitung in Schritt e) die anorganische Phase um 0,6 bis 7 Prozentpunkte, bevorzugt 1,5 bis 3 Prozentpunkte an H$_2$SO$_4$-Konzentration aufkonzentriert wird.

10. Verfahren nach Anspruch 2, dadurch gekennzeichnet, daß zur destillativen Wasserausschleusung so verfahren wird, daß unmittelbar die zum Wiedereinsatz der H$_2$SO$_4$ erforderliche Konzentration und Temperatur eingestellt werden.

**Claims**

1. Process for the continuous or discontinuous preparation of mononitrotoluenes by reaction of toluene with an HNO$_3$/H$_2$SO$_4$/H$_2$O mixture with formation, essentially, of mononitrotoluenes and reaction water, characterized by the steps

a) feeding the reaction participants toluene, HNO$_3$, H$_2$SO$_4$ and H$_2$O in any sequence into a reactor equipped with mixing elements, in which

al) the amount of HNO$_3$ is 1 - 8 % by weight, the amount of H$_2$SO$_4$ is 58 - 74 % by weight and the amount of H$_2$O is the remainder to 100 % by weight and 100 % by weight signifies the sum of HNO$_3$ + H$_2$SO$_4$ + H$_2$O,

a2) the H$_2$O is used as such, as dilution H$_2$O of the HNO$_3$, as dilution H$_2$O of the H$_2$SO$_4$ or in a plurality of the said forms and

a3) the molar ratio of toluene to HNO$_3$ is 0.9 - 1.5,

b) rapid and intensive mixing of the totality of the reaction participants, for which a mixing energy is employed of 1 to 40 watts per litre of the total reaction mixture, preferably 3 to 30 W/l,

c) carrying out the reaction under adiabatic conditions, the reaction participants being fed in at temperatures such that the mixing proceeds in the range of 20 - 110°C, preferably 30 - 100°C, particularly preferably 40 - 90°C, and the temperature at the end of the reaction does not exceed 135°C,

d) separating the reaction mixture, after carrying out the reaction, into an organic and an inorganic phase and

e) work-up of the substantially $HNO_3$-free inorganic phase by distillation with removal of water.

2. Process according to Claim 1, characterized in that the process is carried out continuously and for this tubular reactors are used which substantially prevent the back-mixing of the reaction participants.

3. Process according to Claim 2, characterized in that the reaction participants, before entry into the reactor substantially preventing back-mixing, are intensively mixed with the aid of mixing elements within a time of less than 3 sec. and, on flowing through the reactor, are redispersed at least twice.

4. Process according to Claim 1, characterized in that, in step e), the heat of reaction absorbed by the inorganic phase, as a result of the adiabatic reaction conditions, is utilized for the ejection by distillation of water at a pressure of 1 - 100 mbar, preferably 5 - 80 mbar, particularly preferably 10 - 75 mbar and $H_2SO_4$ is preferably produced in this case which is suitable for use in step a).

5. Process according to Claim 1, characterized in that the sulphuric acid content in the acid phase according to step d) is 62 to 74 % by weight, preferably 64 to 72 % by weight, particularly preferably 66 to 70 % by weight.

6. Process according to Claim 1, characterized in that the content of added nitric acid in the reaction mixture at the time of mixing, based on the sum of nitric acid, sulphuric acid and water, is 1 to 6 % by weight, preferably 1.5 to 4 % by weight.

7. Process according to Claim 1, characterized in that the content of sulphuric acid in the reaction mixture at the time of mixing, based on the sum of nitric acid, sulphuric acid and water, is 60 - 72 % by weight, preferably 61 - 69 % by weight.

8. Process according to Claim 1, characterized in that the molar ratio of toluene to nitric acid is greater than 1.0 to 1.5, preferably 1.03 to 1.3, particularly preferably 1.05 to 1.2.

9. Process according to Claim 1, characterized in that, in the work-up by distillation in step e), the inorganic phase is concentrated in $H_2SO_4$ concentration by 0.6 to 7 percentage points, preferably 1.5 to 3 percentage points.

10. Process according to Claim 2, characterized in that, for the ejection of water by distillation, conditions are employed such that the concentration and temperature required for the reuse of the $H_2SO_4$ are directly established.


**Revendications**

1. Procédé pour la préparation en continu ou en discontinu de mononitrotoluènes par mise en réaction de toluène avec un mélange de $HNO_3/H_2SO_4/H_2O$ avec formation essentiellement des mononitrotoluènes et d'eau réactionnelle, caractérisé par les étapes consistant à :

a) introduire les partenaires réactionnels toluène, $HNO_3$, $H_2SO_4$ et $H_2O$ dans n'importe quel ordre dans un réacteur équipé d'organes de mélange,

a1) la quantité de $HNO_3$ s'élevant à 1-8% en poids, la quantité de $H_2SO_4$ à 58-74% en poids et la quantité de $H_2O$ représentant le reste pour obtenir 100% en poids, 100% en poids représentant la somme de $HNO_3$ + $H_2SO_4$ + $H_2O$,

a2) le $H_2O$ étant mis en oeuvre comme tel, sous forme du $H_2O$ de la dilution du $HNO_3$, sous forme du $H_2O$ de la dilution du $H_2SO_4$ ou encore sous plusieurs des formes mentionnées, et

a3) le rapport molaire du toluène au HNO$_3$ s'élevant de 0,9 à 1,5,

b) mélanger rapidement et intimement la quantité totale des partenaires réactionnels en utilisant pour ce faire une énergie de mélange de 1 à 40 watt par litre du mélange réactionnel total, de préférence de 3 à 30 w/l,

c) effectuer la mise en réaction dans des conditions adiabatiques, les partenaires réactionnels étant introduits à des températures telles que le mélangeage a lieu dans le domaine de 20 à 110°C, de préférence de 30 à 100°C, de manière particulièrement préférée de 40 à 90°C, et la température ne dépassant pas 135°C à la fin de la réaction,

d) séparer le mélange réactionnel après mise en oeuvre de la réaction, en une phase organique et en une phase inorganique, et

e) traiter par distillation la phase inorganique exempte de HNO$_3$ dans une large mesure, tout en éliminant l'eau.

2. Procédé selon la revendication 1, caractérisé en ce qu'on l'effectue en continu et en ce qu'on met en oeuvre, à cet effet, des réacteurs tubulaires qui empêchent, dans une large mesure, le remélangeage des partenaires réactionnels.

3. Procédé selon la revendication 2, caractérisé en ce qu'on mélange intimement, pendant un laps de temps inférieur à 3 secondes, à l'aide d'organes de mélange, les partenaires réactionnels avant leur introduction dans le réacteur empêchant le remélangeage dans une large mesure et on les remet en dispersion au moins à deux reprises après leur passage dans le réacteur.

4. Procédé selon la revendication 1, caractérisé en ce qu'à l'étape e), on utilise la chaleur réactionnelle récupérée de la phase inorganique suite à la mise en réaction adiabatique, pour l'éclusage au-dehors de l'eau par distillation sous une pression de 1-100 mbar, de préférence de 5-80 mbar, de manière particulièrement préférée de 10-75 mbar, et on obtient de préférence en l'occurrence un H$_2$SO$_4$ qui est approprié pour la mise en oeuvre dans l'étape a).

5. Procédé selon la revendication 1, caractérisé en ce que la teneur en acide sulfurique dans la phase acide après l'étape d) s'élève de 62 à 74% en poids, de préférence de 64 à 72% en poids, de manière particulièrement préférée de 66 à 70% en poids.

6. Procédé selon la revendication 1, caractérisé en ce que la teneur du mélange réactionnel en acide nitrique ajouté au moment du mélangeage, rapportée à la somme de l'acide nitrique, de l'acide sulfurique et de l'eau s'élève de 1 à 6% en poids, de préférence de 1,5 à 4% en poids.

7. Procédé selon la revendication 1, caractérisé en ce que la teneur du mélange réactionnel en acide sulfurique au moment du mélangeage, rapportée à la somme de l'acide nitrique, de l'acide sulfurique et de l'eau s'élève de 60 à 72% en poids, de préférence de 61 à 69% en poids.

8. Procédé selon la revendication 1, caractérisé en ce que le rapport molaire du toluène à l'acide nitrique est supérieur à 1,0-1,5, de préférence à 1,03-1,3, de manière particulièrement préférée à 1,05-1,2.

9. Procédé selon la revendication 1, caractérisé en ce que, au cours du traitement par distillation à l'étape e), on augmente dans la phase inorganique la concentration en H$_2$SO$_4$ de 0,6 à 7 points de pourcentage, de préférence de 1,5 à 3 points de pourcentage.

10. Procédé selon la revendication 2, caractérisé en ce qu'on procède, pour l'éclusage de l'eau au-dehors par distillation, de telle sorte qu'on règle immédiatement la concentration et la température requises pour le recyclage du H$_2$SO$_4$.